# EUROPEAN PATENT SPECIFICATION

(11) **EP 2 021 698 B1**
(45) Date of publication and mention of the grant of the patent: **29.07.2015**
(21) Application number: 07729081.5
(22) Date of filing: 14.05.2007
(51) Int. Cl.: B03C 3/16, B03C 3/38, F24F 13/22, F24F 1/02, A61L 9/22, F24F 3/16, B03C 3/017

(54) **AIR CONDITIONING AND CLEANING APPARATUS**
LUFTKLIMATISIERUNGS- UND -REINIGUNGSVORRICHTUNG
APPAREIL DE CLIMATISATION ET DE NETTOYAGE

(30) Priority: 12.05.2006 ES 200601310
(43) Date of publication of application: 11.02.2009
(73) Proprietor: BSH Hausgeräte GmbH, 81739 München (DE)
(72) Inventor: BERAZALUCE MINONDO, Iñigo, 31004 Pamplona (ES)
(86) International application number: PCT/EP2007/054629
(87) International publication number: WO 2007/131981

(56) References cited:
- WO-A-2006/018978
- DE-A1- 10 209 994
- DE-A1- 19 842 068
- US-A- 4 133 652
- US-A- 5 009 683

## Description

The invention relates to an air conditioning and cleaning apparatus which comprises at least one air inlet point, one air outlet point, an ionisation device with a voltage source for ionising the particles contained in the incoming air, a heat exchanger device with at least one electrically charged surface which acts as a surface for collecting ionised particles, and a fan.

In many cases, the air which is present inside work rooms or areas does not satisfy the requirements of the users of said spaces as regards the temperature, the moisture content and the purity of the air. Frequently said air is laden with harmful substances, for example small particles such as dust or cigarette smoke, as well as organic particles such as spores, mites, bacteria or volatile organic compounds.

Different solutions for performing the cleaning and conditioning of the air in a closed space are already known (see for example patent document DE-10209994-A). According to the state of the art air cleaning devices are available, being based, among other methods, on the electrostatic cleaning of the air or cleaning of the air by means of a nanoporous filter.

A device of this type for the electrostatic cleaning of the air contains air ionising electrodes. Ionisation of the air has the effect that the particles of dust which it contains are charged and, thereafter, are collected in a collector device situated along the direction of the air flow. The collector device is composed in this case of a collection surface in the form of metal plates onto which the charged particles adhere. One problem which this type of filter has is that of the systems for cleaning these plates which either have to be disassembled or washed every so often or else require complicated internal washing or vibrating systems for dislodging the accumulated dust. The document DE 198 42 068, for example, describes an electrostatic air cleaning device, by means of which the air which is to be conveyed is ionised and passes to a positive voltage and gas-permeable electrode.

Likewise, the use of nanoporous mechanical filters is known. Nevertheless, said filters tend to become blocked up rapidly with the separated components, owing to the limited size of their mesh aperture. As a result they must be replaced or thoroughly cleaned. Also, a large amount of energy must be used in order to introduce the necessary quantity of air through the filter. Together with the large quantity of energy used, this gives rise also in particular to noise-related problems.

These air cleaning devices are frequently installed in air processing apparatus such as air conditioning apparatus and dehumidifiers. According to the state of the art there exist devices which combine some of the techniques mentioned in this type of air conditioning device.

The document US 4133652 describes an electric air conditioning apparatus in which an electrostatic air cleaning unit is combined with a mechanical filter. In said unit provision was made at the air inlet point for passing through a mechanical filter and, thereafter, the arrangement of the air ionisation device. The air is circulated by means of a propeller which causes it to pass through a heat exchanger unit. This document describes how, when the space is very restricted, for example in motor cars, the vanes of the heat exchanger perform the function of a particle collection surface.

All the techniques available according to the state of the art have the disadvantage that they require exhaustive maintenance. Thus, the surfaces where ionised particles are absorbed must be cleaned frequently, since the absorbed particles may occupy them completely and act as electrical insulators. The mechanical filters tend to become blocked, owing to the small size of their pores, such that they must be replaced or cleaned more frequently.

This is the starting point for the object of the present invention which aims to provide an air conditioning and cleaning apparatus with a self-cleaning filter which operates with a high degree of efficiency and at the same time is very compact and silent, saves a lot of energy and requires as little maintenance as possible.

Said function is achieved by means of a combined air conditioning and cleaning apparatus according to the characteristic features of Claim 1. The dependent claims relate to those configurations of the invention which may be used both separately and in combination with each other.

By means of an air conditioning and cleaning apparatus according to the invention results which are an indication of notable improvements compared to the state of the art are achieved. Regulation, by means of a controller, of the cleaning of the surface of the heat exchange device using condensed water ensures a high level of efficiency in cleaning of the air as well as the need for little maintenance, at the same time as low costs and an apparatus with compact dimensions. Since the heat exchange device acts at the same time as a collection surface for the electrostatically charged particles, the device may be produced in a very low-cost manner and with small dimensions. With the controller of the heat exchange device, the temperature of the heat exchange device may be regulated so that condensed water forms on the surface of said heat exchange device. The water which condenses flows down the surface of the heat exchange device, in this way transporting with it the particles of dust adhering thereto, this having the advantage that the surface collecting the electrostatically charged particles is thus cleaned automatically, making manual cleaning thereof unnecessary. This is important since a layer of electrostatically charged particles formed on the collecting surface acts as an electrical insulator, this causing a reduction in the efficiency of said collecting surface. Thus, cleaning of the collecting surface is indispensable when a layer of particles with an electrostatic charge has adhered to it.

In one embodiment of the apparatus, the heat exchanger device acts at least as an evaporator of a cooling circuit being moreover able to function as a condenser if it operates the circuit as a heat pump.

The controller activates the heat exchanger device, when it is not active as an evaporator, after lapsing of a given operating period of the ionisation device. In the case where the apparatus is functioning as an air cleaner without conditioning or as an air cleaner with heat pump, the controller activates the heat exchanger device as an evaporator so that water condenses on the surface and it is cleaned automatically after a certain operating period of the ionisation device, performing cleaning cycles. Thus the air cleaning efficiency of the apparatus is increased.

Moreover, the apparatus may have a contamination sensor which sends a signal to the controller in order to activate the heat exchanger device when it is not active as an evaporator, upon detection of a given level of contamination in the air which enters into the apparatus and/or on the surface of the set and/or in the air which has passed through the set. Thus, provided that it detects a high degree of contamination in the environment or on the surface of the heat exchanger device or if it does not filter correctly the air passing through it, it will activate a cleaning cycle. Also it could regulate the time between cleaning cycles, taking into account the quantity of particles eliminated from the air based on the sensor measurement.

The apparatus may also comprise a fan for forcing an air flow from the air inlet point to the air outlet point. In this way, in spaces where there is no movement of the air, it may be forced to pass through the apparatus. The controller regulates the air flow to ensure that said air reaches the dew point and condenses water on the surface of the heat exchanger device. Thus the controller may accelerate or slow down the passage of air through the heat exchanger device. The lower the degree of ambient humidity of the air in the location to be conditioned, namely the drier the environment, the more the through-flow will be slowed down in order to condense water on the surface of the heat exchanger device and perform the cleaning cycle.

Preferably the distance between the ionisation device and the surface of the exchanger device is between 7 and 30 mm. In this way the ionised particles will be attracted more strongly by the collecting surface of the heat exchanger device.

In order to guarantee the safety of the device, when the surface of the exchanger device is connected to earth, it is ensured that the current flowing through the ioniser is between 0 and 5 mA, this value being able to be reduced in the case of more stringent requirements. In this way, and despite the small distance between collecting surface and ioniser, the safety of the device is ensured even if they should come into contact.

In accordance with another of the advantageous embodiments, the ionisation device has wires, and/or meshes, and/or projections. In said embodiment the arrangement of numerous wires, or the arrangement thereof in the form of meshes, is especially advantageous. This ensures that, in the event of a wire blowing, a short-circuit will not occur.

The electric wires and/or meshes and/or projections of the ionisation device are preferably made of stainless steel and/or copper.

Likewise, it is advantageous to use wires and/or meshes and/or projections, the diameter of which is between 0.05 and 0.5 mm, preferably between 0.07 and 0.3 mm. Especially advantageous are dimensions of between 0.08 and 0.2 mm, preferably 0.1 mm. By using these small-length diameters for the wires and/or the meshes and/or the projections it is possible to achieve a greater degree of efficiency.

According to another of the preferred embodiments, the ionisation device and the heat exchange device are insulated or situated separated from each other such that the electrical resistance is increased to between 5 and 50 megaohms, preferably between 10 and 40 megaohms, a resistance of between 15 and 30 megaohms, and preferably 20 megaohms, being especially advantageous.

By means of the air conditioning and cleaning apparatus according to the invention, notable improvements compared to the state of the art are achieved.

Other advantages and constructional features of the invention are described below and others may be deduced with reference to the accompanying drawings and by means of the examples of embodiment.

The drawings show in schematic form:
- Fig. 1.-: a schematic representation of a preferred embodiment of an air conditioning and purification apparatus such as that according to the invention.

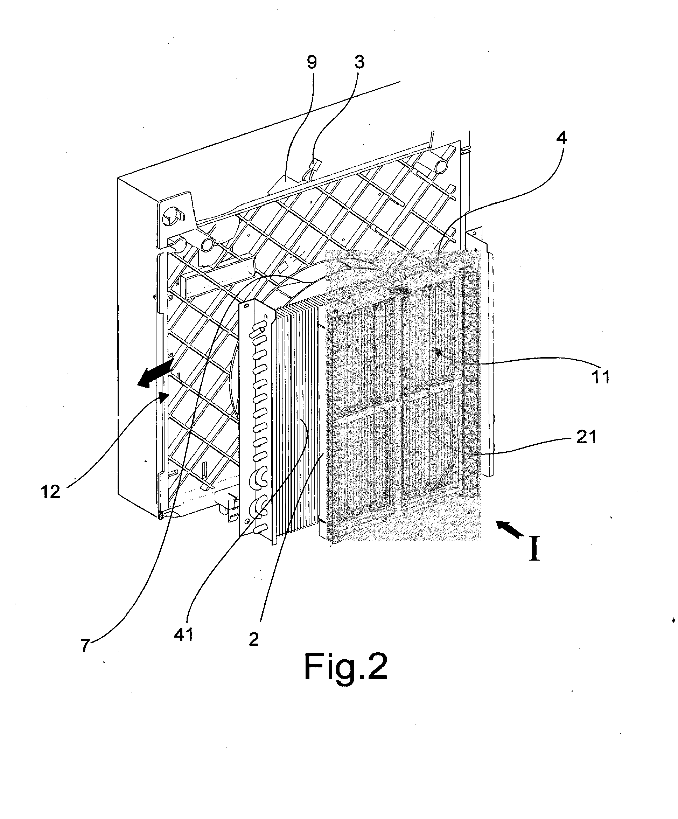

- Fig. 2.-: an angled perspective view from above of an ionisation device facing the heat exchange device and the fan.
- Fig. 3.-: a perspective view of a heat exchange device, situated at a distance from an ionisation device which is connected to a voltage source.
- Fig. 4.-: a heat exchanger device.

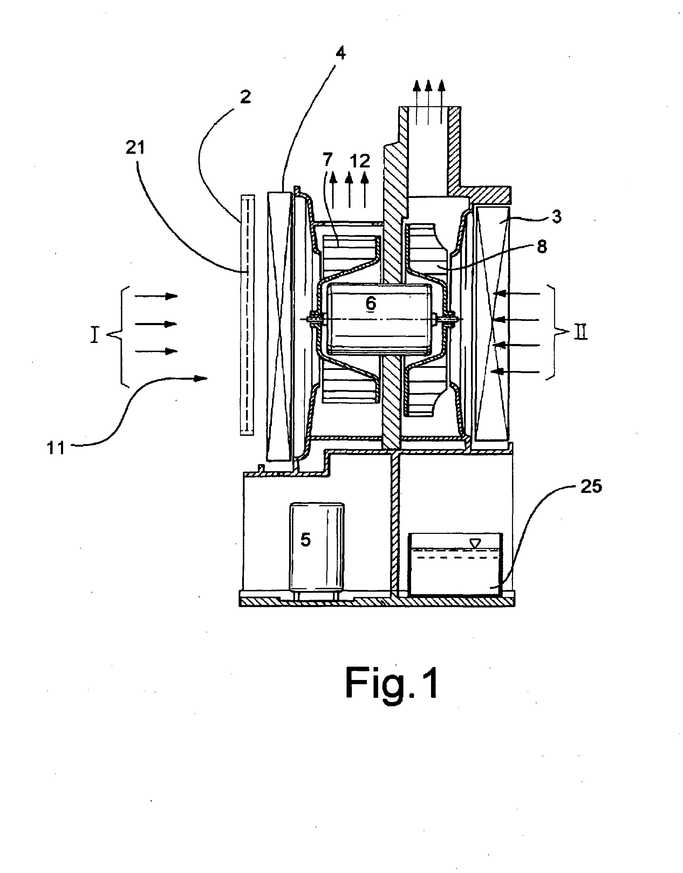

Figure 1 shows a schematic illustration of a preferred embodiment of an air conditioning and purification apparatus, such as that of the invention, which comprises two heat exchanger devices: a first exchanger device which functions as an evaporator 4 and a second exchanger functioning as a condenser 3 connected in series in a common cooling circuit (not shown in the figures). For conveying of a coolant the cooling circuit has a compressor 5. This cooling circuit may be reversed and change its operating mode so as to operate as a heat pump, namely the evaporator functions as a condenser and vice versa. The apparatus described is a portable apparatus without an external unit. It could likewise comprise an external unit where the second heat exchanger device would be situated. In the case of the apparatus according to Figure 1, by means of a fan 7 operated by a motor 6, a first flow I of air is conveyed from the environment and made to pass through the ionisation device 2, where the particles of dust contained in it are electrically charged, and through the evaporator 4, as indicated by the direction of the arrows. The first flow I of air from the surrounding environment is cooled as it passes through the evaporator 1 and returned into the space which is to be conditioned.

Through the second heat exchange device 3 a second flow II of air is conveyed from the environment, being moved by the fan 8 operated by the same motor 6 which operates the fan 7. This second flow II of air from the environment is heated owing to the high temperatures of the condenser 3 when it is operating as an air conditioning apparatus. As a result the second flow II of air from the environment has the function of reducing the heat of the condenser 3 and is conveyed to the exterior of the apparatus, and not into the space which is to be conditioned, being instead conveyed as exhaust air into the external environment. In the case where the apparatus is to be used as a dehumidifier, the exhaust air of both the flows would be conveyed into the space to be conditioned.

Figure 1 shows how the air flow I enters via an air inlet point and circulates first through an ionisation device. By means of a voltage source a voltage of between 6,000 and 12,000 volts is applied to the ionisation device and this causes the particles contained in the air flow to be electrically charged. The ionisation device is composed of wires, but said device may also be composed of meshes or projections (not shown in the figures). The wires of the ionisation device preferably have a thickness of 0.1 mm. With the aim of preventing parasitic induction during ionisation, and not solely for safety purposes, the distance a between the heat exchanger device and the ionisation device is fixed at about 12 mm so that the electrical resistance increases to 20 megaohms. After passing through the ionisation device the air reaches the first heat exchange device operating as an evaporator. The surface of the evaporator is made of a material which is an electricity conductor and the ionised particles which are present in the air adhere to its surface.

In order to guarantee the safety of the apparatus, when the surface of the exchanger device 4 is connected to earth, it is ensured that the flow circulating through the ioniser is between 0 and 5 mA, this value being able to be reduced in the case of more stringent requirements. In this way, and despite the small distance between the collecting surface and ioniser, the safety condition of the device is ensured even if they should come into contact or an electric arc should form between them.

When the air conditioning apparatus is functioning, the evaporator 1 is at a low temperature. The air I of the environment is charged with ambient moisture. This moisture is condensed upon passing through the evaporator and droplets of condensation water form on the surface of the evaporator and flow down and trickle into a collection basin arranged underneath it. These water droplets transport the particles which are present on the surface of the evaporator and clean it.

After circulation of the air through the evaporator, the air flow continues to flow towards the outlet, forced by the fan 7. In this way a continuous air flow is ensured during operation according to the invention. Finally, the purified air returns into the room through an air outlet point.

In order to keep the surface of the heat exchange device 4 clean, it is necessary for sufficient water to condense on it and transport the particles. To ensure that condensation is produced, the apparatus according to the invention comprises a controller 9. This controller regulates operation of the heat exchanger device 4. It is able to do this in many ways, for example, it counts the operating time of the ionisation device 2 and when an operating time t of the ioniser 2 has lapsed, it activates the cold circuit so that the heat exchanger device 4 acts as an evaporator for a time period t2 so that sufficient water is condensed on the surface 41 of the evaporator. Once the time period t2 has lapsed, the time count is restarted in order to perform another cleaning cycle.

The apparatus may also comprise a contamination sensor for the air entering the apparatus. This sensor sends the signal to the controller 9, and the latter, depending on the degree of contamination of the air, performs a cleaning cycle. Also it is possible to have another contamination sensor for the evaporator surface. When this sensor detects a certain level of dust on the surface 41 of the evaporator, it sends a signal to the controller 9 so that it performs a cleaning cycle. Another possible sensor may be situated at the outlet 12 for the air which has passed through the evaporator 4, sending a signal as to the particles contained in the expelled air to the controller, which starts the cleaning cycle if necessary.

It may be the case that the degree of ambient moisture is very small. To ensure that water condenses in the evaporator 4, the controller 9 regulates the speed of the air flow I which circulates through the evaporator via the fan 7. The slower the circulation of the air flow, the greater the amount of condensation.

In this way, it is possible to use the apparatus solely as an air purifier ensuring self-cleaning of the dust collector since the controller 9 will cause activation of the cold circuit in order to perform the necessary cleaning cycles. The same will happen when it functions as an air purifier and heat pump since, if the heat exchanger device 4 is functioning as a condenser, it accumulates the ionised particles on its surface 41 but does not condense the water. Upon lapsing of a given time t without the heat exchanger device 4 functioning as an evaporator, the controller 9 will reverse for a time period t2 operation of the cooling circuit and will cause operation of the heat exchanger device as an evaporator so that it performs at least one cleaning cycle.

It is envisaged that the water which condenses on the surface of the evaporator 4 is collected inside a storage vessel 25 so that the user can remove it when it is full. Also it is possible to provide a filter for water so as to cause it to recirculate through the second exchanger device 3 when it is operating in the manner of a condenser so as to cool it.

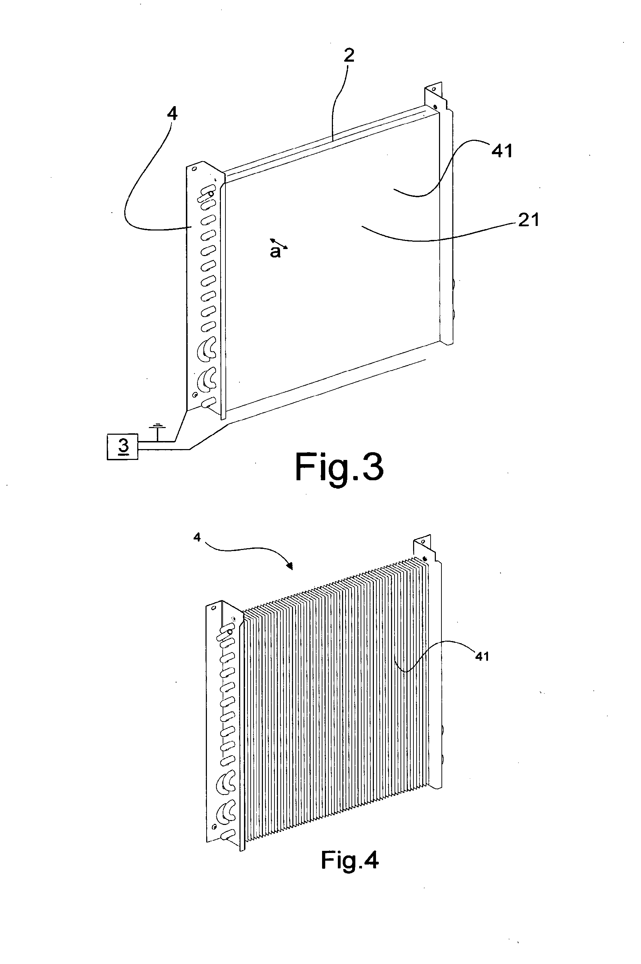

Figure 3 shows a perspective view of a heat exchanger device 4, situated at a distance from an ionisation device 2. The ionisation device is connected to a voltage source 3 and has, by way of example, wires between which the air circulates. The ionisation device is electrically charged so that the heat exchange unit attracts the particles which are ionised by the ionisation device, which remain attached to said heat exchange unit. When this occurs, the temperature of the heat exchange device is regulated so that water condenses on the surface of the heat exchange device, said water flowing so as to clean said surface.

Figure 4 shows a heat exchanger device 4 and 3 of the type which comprises a plurality of metal heat-radiating fins 41 arranged in parallel. It is especially advantageous if they are arranged totally vertical so that the condensed water flows off more easily and conveys with it all the impurities which are present on the surfaces 41. However, they could also be inclined so as to prevent the stagnation of water on top of them.

By means of the air conditioning and cleaning device according to the invention for the first time the result is achieved that air is purified in an efficient and low-cost manner and at the same time the dimensions of the device are smaller.

## Claims

1. Air conditioning and cleaning apparatus which comprises at least one air inlet point (11), one air outlet point (12), an ionisation device (2) for ionising the particles contained in the incoming air and at least one heat exchanger device (4) with at least one surface (41) which acts as a surface for collecting ionised particles, **characterised in that** the apparatus comprises a controller (9) which regulates operation of the heat exchanger device (4) so that condensed water forms on the surface (41) of said heat exchanger device and cleans said surface of the heat exchanger device, preventing saturation with particles collected by the surface of the heat exchanger device.

2. Air conditioning and cleaning apparatus according to Claim 1, **characterised in that** the heat exchanger device (4) acts at least as an evaporator of a cooling circuit.

3. Air conditioning and cleaning apparatus according to Claim 2, **characterised in that** the controller (9) activates the heat exchanger device (4), when it is not active as an evaporator, after lapsing of a given operating time (t) of the ionisation device (2).

4. Air conditioning and cleaning apparatus according to Claim 2 or 3, **characterised in that** it comprises a contamination sensor which sends a signal to the controller (9) in order to activate the heat exchange device (4), when it is not active as an evaporator, upon detection of a given level of contamination of the air (I) which enters the apparatus and/or on the surface of the set and/or in the air which has passed through the heat exchanger device.

5. Air conditioning and cleaning apparatus according to the preceding claims, **characterised in that** it comprises a fan (7) for forcing an air flow from the air inlet point (11) to the air outlet point (12).

6. Air conditioning and cleaning apparatus according to Claim 5, **characterised in that** controller (9) regulates the air flow so as to ensure that said air reaches the dew point and condenses water on the surface of the heat exchanger device (4).

7. Air conditioning and cleaning apparatus according to the preceding claims, **characterised in that** the distance (a) between the ionisation device (2) and the surface of the heat exchanger device (4) is between 7 and 30 mm.

8. Air cleaning device according to the preceding claims, **characterised in that** the ionisation device (2) has wires (21), meshes and/or projections.

9. Apparatus according to the claim, **characterised in that** the electric wires (21) and/or the meshes and/or the projections of the ionisation device (2) are made of stainless steel and/or copper.

10. Apparatus according to Claims 8 or 9, characterised it that the diameter of the wires (21) and/or the meshes and/or the projections measures between 0.05 and 0.5 mm, preferably between 0.07 and 0.3 mm, the dimensions of between 0.08 and 0.2 mm, in particular, 0.1 mm, being especially advantageous.

11. Apparatus according to one of the preceding claims, **characterised in that** the ionisation device (2) and the heat exchanger device (4) are insulated or arranged separated from each other so that the electrical resistance reaches a value of between 5 and 50 megaohms, preferably between 10 and 40 megaohms, a value of between 15 and 30 megaohms, in particular 20 megaohms, being especially advantageous.

12. Air cleaning apparatus according to the preceding claims, **characterised in that** the maximum electric current which flows between the ionisation device (2) and the heat exchanger device (4) is limited to 5 mA in order to ensure the safety of the device.

## Patentansprüche

1. Luftkonditionierungs- und -reinigungsvorrichtung, die mindestens eine Lufteinlassstelle (11), eine Luftauslassstelle (12), eine Ionisiereinrichtung (2) zum Ionisieren der Partikel in der hereinströmenden Luft und mindestens eine Wärmetauschereinrichtung (4) mit mindestens einer Fläche (41) umfasst, die als Fläche zum Auffangen ionisierter Partikel dient, **dadurch gekennzeichnet, dass** die Vorrichtung eine Steuerung (9) umfasst, die den Betrieb der Wärmetauschereinrichtung (4) so regelt, dass sich auf der Fläche (41) der Wärmetauschereinrichtung Kondenswasser bildet und die Fläche der Wärmetauschereinrichtung reinigt, was eine Sättigung mit von der Fläche der Wärmetauschereinrichtung aufgefangenen Partikeln verhindert.

2. Luftkonditionierungs- und -reinigungsvorrichtung nach Anspruch 1, **dadurch gekennzeichnet, dass** die Wärmetauschereinrichtung (4) zumindest als Verdampfer eines Kühlkreislaufs dient.

3. Luftkonditionierungs- und -reinigungsvorrichtung nach Anspruch 2, **dadurch gekennzeichnet, dass** die Steuerung (9) nach Ablauf einer vorgegebenen Betriebsdauer (t) der Ionisiereinrichtung (2) die Wärmetauschereinrichtung (4) aktiviert, wenn diese nicht als Verdampfer fungiert.

4. Luftkonditionierungs- und -reinigungsvorrichtung nach Anspruch 2 oder 3, **dadurch gekennzeichnet, dass** sie einen Verschmutzungssensor umfasst, der bei Erkennen eines bestimmten Verschmutzungspegels der Luft (I), die in die Vorrichtung strömt, und/ oder auf der Fläche der Einheit und/ oder in der Luft, die die Wärmetauschereinrichtung durchströmt hat, ein Signal zur Steuerung (9) sendet, um die Wärmetauschereinrichtung (4) zu aktivieren, wenn diese nicht als Verdampfer fungiert.

5. Luftkonditionierungs- und -reinigungsvorrichtung nach den vorhergehenden Ansprüchen, **dadurch gekennzeichnet, dass** sie einen Ventilator (7) zum Forcieren einer Luftströmung von der Lufteinlassstelle (11) zur Luftauslassstelle (12) umfasst.

6. Luftkonditionierungs- und -reinigungsvorrichtung nach Anspruch 5, **dadurch gekennzeichnet, dass** die Steuerung (9) die Luftströmung so regelt, dass die Luft den Taupunkt erreicht und Wasser auf der Fläche der Wärmetauschereinrichtung (4) kondensiert.

7. Luftkonditionierungs- und -reinigungsvorrichtung nach den vorhergehenden Ansprüchen, **dadurch gekennzeichnet, dass** der Abstand (a) zwischen der Ionisiereinrichtung (2) und der Fläche der Wärmetauschereinrichtung (4) zwischen 7 und 30 mm beträgt.

8. Luftreinigungseinrichtung nach den vorhergehenden Ansprüchen, **dadurch gekennzeichnet, dass** die Ionisiereinrichtung (2) Drähte (21), Maschen und/ oder Vorsprünge aufweist.

9. Vorrichtung nach dem Anspruch, **dadurch gekennzeichnet, dass** die elektrischen Drähte (21) und/ oder die Maschen und/ oder die Vorsprünge der Ionisiereinrichtung (2) aus rostfreiem Stahl und/ oder Kupfer hergestellt sind.

10. Vorrichtung nach Anspruch 8 oder 9, **dadurch gekennzeichnet, dass** der Durchmesser der Drähte (21) und/ oder der Maschen und/ oder der Vorsprünge zwischen 0,05 und 0,5 mm, vorzugsweise zwischen 0,07 und 0,3 mm beträgt, wobei die Abmessungen zwischen 0,08 und 0,2 mm, insbesondere 0,1 mm, besonders vorteilhaft sind.

11. Vorrichtung nach einem der vorhergehenden Ansprüche, **dadurch gekennzeichnet, dass** die Ionisiereinrichtung (2) und die Wärmetauschereinrichtung (4) voneinander isoliert oder getrennt angeordnet sind, so dass der elektrische Widerstand einen Wert zwischen 5 und 50 Megaohm, vorzugsweise zwischen 10 und 40 Megaohm erreicht, wobei ein Wert zwischen 15 und 30 Megaohm, insbesondere 20 Megaohm, besonders vorteilhaft ist.

12. Luftreinigungsvorrichtung nach den vorhergehenden Ansprüchen, **dadurch gekennzeichnet, dass** der maximale elektrische Strom, der zwischen der Ionisiereinrichtung (2) und der Wärmetauschereinrichtung (4) fließt, zur Gewährleistung der Sicherheit der Einrichtung auf 5 mA begrenzt ist.

## Revendications

1. Appareil de climatisation et de purification d'air qui comprend au moins un point d'entrée d'air (11), un point de sortie d'air (12), un dispositif d'ionisation (2) pour ioniser les particules contenues dans l'air entrant et au moins un dispositif échangeur de chaleur (4) avec au moins une surface (41) qui agit comme une surface pour collecter des particules ionisées, **caractérisé en ce que** l'appareil comprend un contrôleur (9) qui régule le fonctionnement du dispositif échangeur de chaleur (4) de façon à ce que de l'eau condensée se forme sur la surface (41) dudit dispositif échangeur de chaleur et purifie ladite surface du dispositif échangeur de chaleur, empêchant une saturation avec des particules collectées par la surface du dispositif échangeur de chaleur.

2. Appareil de climatisation et de purification d'air selon la revendication 1, **caractérisé en ce que** le dispositif échangeur de chaleur (4) agit au moins comme un évaporateur d'un circuit de refroidissement.

3. Appareil de climatisation et de purification d'air selon la revendication 2, **caractérisé en ce que** le contrôleur (9) active le dispositif échangeur de chaleur (4), lorsqu'il n'est pas actif comme un évaporateur, après écoulement d'un temps de fonctionnement (t) donné du dispositif d'ionisation (2).

4. Appareil de climatisation et de purification d'air selon la revendication 2 ou 3, **caractérisé en ce qu'**il comprend un capteur de contamination qui envoie un signal au contrôleur (9) afin d'activer le dispositif échangeur de chaleur (4), lorsqu'il n'est pas actif comme un évaporateur, à la détection d'un niveau donné de contamination de l'air (I) qui pénètre dans l'appareil et/ou sur la surface de l'ensemble et/ou dans l'air qui est passé à travers le dispositif échangeur de chaleur.

5. Appareil de climatisation et de purification d'air selon les revendications précédentes, **caractérisé en ce qu'**il comprend un ventilateur (7) pour forcer un écoulement d'air du point d'entrée d'air (11) jusqu'au point de sortie d'air (12).

6. Appareil de climatisation et de purification d'air selon la revendication 5, en ce que le contrôleur (9) régule l'écoulement d'air de façon à garantir que atteint le point de rosée et condense de l'eau sur la surface du dispositif de chaleur (4).

7. Appareil de climatisation et de purification d'air selon les revendications précédentes, **caractérisé en ce que** la distance (a) entre le dispositif d'ionisation (2) et la surface du dispositif échangeur de chaleur (4) est entre 7 et 30 mm.

8. Appareil de purification d'air selon les revendications précédentes, **caractérisé en ce que** le dispositif d'ionisation (2) a des fils (21), des mailles et/ou des projections.

9. Appareil selon la revendication, **caractérisé en ce que** les fils électriques (21) et/ou les mailles et/ou les projections du dispositif d'ionisation (2) sont constituée(s) d'acier inoxydable et/ou de cuivre.

10. Appareil selon les revendications 8 ou 9, **caractérisé en ce que** le diamètre des fils (21) et/ou des mailles et/ou des projections mesure entre 0,05 et 0,5 mm, préférablement entre 0,07 et 0,3 mm, les dimensions d'entre 0,08 et 0,2 mm, en particulier 0,1 mm, étant spécialement avantageuses.

11. Appareil selon l'une des revendications précédentes, **caractérisé en ce que** le dispositif d'ionisation (2) et le dispositif échangeur de chaleur (4) sont isolés ou agencés séparés l'un de l'autre de façon à ce que la résistance électrique atteigne une valeur d'entre 5 et 50 mégaohms, préférablement entre 10 et 40 mégaohms, une valeur d'entre 15 et 30 mégaohms, en particulier 20 mégaohms, étant spécialement avantageuse.

12. Appareil de purification d'air selon les revendications précédentes, **caractérisé en ce que** le courant électrique maximum qui passe entre le dispositif d'ionisation (2) et le dispositif échangeur de chaleur (4) est limité à 5 mA afin de garantir la sûreté du dispositif.
